# FASCICULE DE BREVET EUROPEEN

(11) **EP 1 086 953 B1**
(45) Date de publication et mention de la délivrance du brevet: **23.04.2003**
(21) Numéro de dépôt: 00402414.7
(22) Date de dépôt: 01.09.2000
(51) Int. Cl.: C07H 17/07

(54) **Procédé de purification de la diosmine**
Verfahren zur Reinigung von Diosmin
Process for the purification of diosmin

(30) Priorité: 24.09.1999 FR 9911964
(43) Date de publication de la demande: 28.03.2001
(73) Titulaire: ISOCHEM, 75194 Paris Cedex 04 (FR)
(72) Inventeur: Ferruccio, Laurence, 91810 Vert-Le-Grand (FR); Gibert, Dominique, 60340 Villiers Sous Saint Leu (FR); Senet, Jean-Pierre, 77760 Buthiers (FR); Vergne, Guyselaine, 91720 Maisse (FR)
(74) Mandataire: Pech, Bernard

(56) Documents cités:
- EP-A- 0 860 443
- FR-A- 2 402 655
- FR-A- 2 760 015

## Description

La présente invention se situe dans le domaine pharmaceutique et concerne la diosmine, principe actif médicamenteux utilisé en cas de maladie veineuse ou pour limiter les risques phlébo-vasculaires.
Elle a plus précisément pour objet un procédé de purification de la diosmine.
De façon bien connue de l'homme du métier, la diosmine est généralement synthétisée à partir d'hespéridine au moyen d'une halogénation par l'iode ou le brome, suivie d'une deshydrohalogénation. Le brevet FR 2 402 655 décrit par exemple un tel procédé de préparation de la diosmine par déshydrogénation de l'hespéridine par de l'iode élémentaire.
Tous ces procédés induisent des impuretés iodées ou bromées dans le produit final isolé. Actuellement, les opérations industrielles usuelles conduisent à l'obtention de diosmine possédant un taux d'iode ou de brome résiduel généralement compris entre 500 et 1500 ppm. Par ailleurs, le taux de cendres sulfuriques résiduelles est généralement compris entre 0,2% et 0,5%.
Si de telles teneurs ont longtemps été tolérées par l'industrie pharmaceutique, on s'oriente à l'heure actuelle vers des normes de pharmacopée beaucoup plus exigeantes et draconiennes, à savoir une teneur en halogénures non alimentaires résiduels (I, Br) inférieure à 200 ppm et une teneur en cendres sulfuriques résiduelles inférieure à 0,2%. L'homme du métier est donc à la recherche d'un procédé simple et peu coûteux de purification de la diosmine permettant d'abaisser la teneur en halogénures non alimentaires à une valeur inférieure à 200 ppm, et si possible, d'abaisser également la teneur en cendres sulfuriques résiduelles à une valeur inférieure à 0,2%.

La présente invention a pour objet un tel procédé. Ce procédé permet de passer avec un rendement satisfaisant d'un taux initial élevé en halogénures non alimentaires, par exemple compris entre 500 et 1500 ppm, à une teneur inférieure à 200 ppm.

Il a été découvert, de façon inattendue, que l'on pouvait atteindre d'aussi faibles teneurs en halogénures non alimentaires en faisant réagir dans une première étape, dans un milieu aqueux, la diosmine à purifier avec une base minérale de façon à transformer toute la diosmine en phénate soluble dans le milieu réactionnel et en ajoutant du zinc pulvérulent, puis, dans une deuxième étape, en filtrant le milieu réactionnel et enfin, dans une troisième étape, en acidifiant le filtrat recueilli dans la seconde étape de façon à faire précipiter la diosmine que l'on récupère ensuite de façon usuelle. La diosmine récupérée présente alors un taux d'iode ou de brome résiduel inférieur à 200 ppm.

On a constaté, avec surprise, que seule l'utilisation du zinc permet d'atteindre un tel résultat. Des essais comparatifs réalisés avec d'autres métaux ne permettent pas d'obtenir l'effet technique recherché, à savoir atteindre un taux d'halogénures non alimentaires inférieur à 200 ppm.

Selon une variante préférée de l'invention, la base minérale utilisée lors de la première étape est choisie dans le groupe constitué par la soude, la potasse et leurs mélanges.

De façon particulièrement préférée, on utilise la soude.

La quantité de base minérale introduite est telle que toute la diosmine à purifier est transformée en phénate. Pour se faire, on utilise un excès par exemple compris entre 1,05 et 2 par rapport aux fonctions phénoliques de la diosmine.

Par ailleurs, le zinc pulvérulent peut être ajouté dans le milieu aqueux à tout moment lors de la première étape, à savoir avant, pendant ou après la réaction de la diosmine à purifier avec la base minérale.
De façon particulièrement préférée, la réaction de la diosmine à purifier avec la base minérale a lieu en présence de zinc pulvérulent. Pour ce faire, on ajoute le zinc dans le milieu avant ou au cours de cette réaction.
On préfère particulièrement que le zinc soit présent avant le début de la réaction.
Selon cette variante, on préfère ajouter la base minérale au mélange zinc-diosmine.
La teneur en zinc est en général comprise entre 0,5% en poids et 5% en poids par rapport à la diosmine à purifier, de préférence entre 1% et 3%.
La granulométrie du zinc est en général comprise entre 0,5 et 20µm, de préférence entre 2 et 10µm.

Lors de la première étape, le temps de réaction est en général compris entre 0,5 h et 10 h, de préférence entre 1 h et 8 h, et mieux encore entre 2 h et 6 h.
La température du milieu réactionnel lors de la première étape est comprise entre 0°C et 60°C et, de préférence, entre 20°C et 50°C.

Lors de la troisième étape, le filtrat recueilli est acidifié. Pour ce faire, on peut notamment utiliser des acides organiques tels que l'acide citrique ou l'acide tartrique, ou des acides minéraux tels que l'acide chlorhydrique ou l'acide sulfurique.
De préférence, ces acides sont utilisés en solution aqueuse, à une concentration massique préférentiellement comprise entre 15% et 30%.

Selon une autre variante préférée de l'invention, on utilise un acide de pka ≤ 3. On a constaté que l'utilisation d'un tel acide permet en outre d'abaisser de façon inattendue le taux de cendres sulfuriques résiduelles à une valeur inférieure à 0,2%.
De façon particulièrement préférée, l'acide utilisé est choisi dans le groupe constitué par l'acide chlorhydrique, l'acide sulfurique et leurs mélanges, et, mieux encore, l'acide utilisé est l'acide sulfurique.

L'acide est introduit en quantité telle que toute la diosmine puisse précipiter dans le réacteur. Pour se faire, on utilise un rapport stoechiométrique supérieur à un, par exemple compris entre 1,02 et 2 par rapport à la base minérale.
La température du milieu réactionnel, lors de cette troisième étape, est comprise entre 0°C et 60°C et, de préférence, entre 20°C et 50°C.

Les exemples non limitatifs suivants illustrent l'invention et les avantages qu'elle procure.

### Exemple 1 : Purification de diosmine à l'échelle laboratoire.

### Acidification par H₂SO₄ à 20%.

On met en suspension 60 g (0,0987 mol) de diosmine à purifier dans 100 ml d'eau distillée. Cette diosmine possède un taux d'iode résiduel de 1500 ppm et un taux de cendres sulfuriques de 0,4%. Le taux d'iode résiduel est dosé par ionisation par torche à plasma suivie d'une détection par spectrométrie de masse (IPC.MS) et le taux de cendres sulfuriques, représentatif des sels minéraux présents, est déterminé par analyse gravimétrique : par chauffage en présence d'acide sulfurique à 96%, les matières minérales sont transformées en sulfates appelés « cendres sulfuriques ».
On ajoute 1,3 g de zinc pulvérulent de granulométrie moyenne 3,6µm, soit 2,2% en poids par rapport à la diosmine à purifier. On ajoute 9,9 g (0,247 mol) de soude en solution dans 143 ml d'eau, soit un excès de 1,25 par rapport aux fonctions phénoliques de la diosmine. Lorsque toute la solution aqueuse de soude est ajoutée, on élève la température à 35°C et on maintient cette température pendant 4 h, tout en agitant le milieu. On filtre ensuite le milieu réactionnel sur précouche et on dilue le filtrat avec 100 ml d'eau distillée.
On introduit par ailleurs dans un réacteur de 1 litre, 72 ml d'eau distillée et 19,2 g d'acide sulfurique concentré à 95% en poids (0,186 mol), soit une solution d'acide ayant une concentration massique de 20%. On chauffe à 35°C puis on ajoute le filtrat dilué obtenu précédemment. La diosmine précipite dans le réacteur. On maintient la température à 35°C pendant 30 minutes, puis on refroidit jusqu'à 23°C. On filtre le précipité. Le gâteau recueilli sur le filtre est ensuite rincé avec de l'eau distillée, réempâté dans 300 ml d'eau à 35°C puis filtré et rincé à nouveau.
La diosmine obtenue est séchée en étuve ventilée à 50°C. Après séchage, on obtient 54 g de diosmine. Le rendement de purification est de 90%. La diosmine obtenue présente un taux d'iode résiduel d'environ 75 ppm et un taux de cendres sulfuriques d'environ 0,11%. Les mêmes méthodes de dosage que pour le produit brut ont été utilisées.

### Exemple 2 : Purification de diosmine à l'échelle pilote

### Acidification par H₂SO₄ à 26,5%.

*Première étape* : on introduit dans un réacteur de 30 1 8,09 kg de diosmine humide, soit 5,1 kg de diosmine sèche (8,38 mol) et 10 kg d'eau distillée. La diosmine à purifier possède un taux d'iode résiduel de 1240 ppm (méthode par HPLC ionique) et un taux de cendres sulfuriques de 0,75% (même méthode que pour l'exemple 1).
On ajoute 0,13 kg de zinc pulvérulent de granulométrie moyenne 3,6µm, soit 2,6% en poids par rapport à la diosmine sèche à purifier. On ajoute ensuite 1 kg de soude (25 mol) en solution dans 14,3 kg d'eau distillée, soit un excès de 1,5 par rapport aux fonctions phénoliques de la diosmine. On ajoute la solution aqueuse de soude à l'aide d'une pompe. Quand toute la soude est ajoutée, on élève la température à 35°C et on maintient cette température pendant 4 h, tout en agitant le milieu.

*Deuxième étape* : on filtre ensuite le milieu réactionnel puis on dilue le filtrat avec de l'eau distillée. On obtient un filtrat dilué dont la masse est 49,4 kg.

*Troisième étape* : on introduit dans un réacteur de 25 1 1,96 kg d'eau et 0,75 kg d'acide sulfurique à 95,9% (7,34 mol), soit une solution d'acide ayant une concentration massique en acide sulfurique de 26,5%. On chauffe le milieu à 35°C et on introduit 19,4 kg du filtrat dilué obtenu lors de la deuxième étape à l'aide d'une pompe doseuse. La diosmine précipite dans le réacteur.
En fin d'introduction du filtrat dans le réacteur, on maintient la température à 35°C pendant 30 minutes. On refroidit ensuite le milieu à 25°C. Le refroidissement dure 2,5 h et est effectué sous agitation.
On filtre ensuite le précipité, on rince le gâteau avec de l'eau distillée puis on le réempâte dans 6 kg d'eau à 20°C que l'on chauffe ensuite à 35°C. On filtre et on rince le gâteau.
La diosmine obtenue est séchée 5 h à 50°C en étuve ventilée puis 20 h sous vide partiel de 10mm Hg. Après séchage, on obtient 4,48 kg de diosmine. Le rendement de purification est de 88%. La diosmine obtenue présente un taux d'iode résiduel de 20 ppm et un taux de cendres sulfuriques résiduelles de 0,07%, déterminés selon les mêmes méthodes que pour le produit brut.

### Exemple 3 : Purification de diosmine à l'échelle laboratoire.

### Acidification par HCl.

On met en suspension 60,8 g (0,1 mol) de diosmine à purifier dans 100 ml d'eau distillée. Cette diosmine possède un taux d'iode résiduel de 1350 ppm et un taux de cendres sulfurique de 0,45% (mêmes méthodes de dosage que pour l'exemple 1).
On ajoute 0,69 g de zinc pulvérulent, soit 1,1% en poids par rapport à la diosmine à purifier. On ajoute 9,9 g (0,247 mol) de soude en solution dans 148 ml d'eau, soit un excès de 1,235 par rapport aux fonctions phénoliques de la diosmine. L'addition de la solution de soude se fait à 35°C pendant 30 minutes. Lorsque toute la solution aqueuse de soude est ajoutée, on maintient la température à 35°C pendant 8 h, tout en agitant le milieu.
On filtre ensuite le milieu réactionnel puis on récupère le filtrat après rinçage du filtre avec 140 ml d'eau distillée.

On introduit par ailleurs dans un réacteur 74 g d'une solution aqueuse d'acide chlorhydrique à 12,8% en poids (0,255 mol).
On ajoute le filtrat obtenu précédemment. La diosmine précipite dans le réacteur. On filtre le précipité. Le gâteau recueilli sur le filtre est ensuite lavé avec 120 ml d'eau puis réempâté dans 300 ml d'eau à 35°C pendant 1 heure et filtré et lavé à nouveau avec 120 ml d'eau.
La diosmine obtenue est séchée à 50°C sous vide partiel de 10mm Hg. Après séchage, on obtient 55,3 g de diosmine. Le rendement de purification est de 91%. Elle présente un taux d'iode résiduel de 100 ppm et un taux de cendres sulfuriques de 0,06%. Les mêmes méthodes de dosage que pour le produit brut ont été utilisées.

### Exemple 4 : Purification de diosmine à l'échellelaboratoire.

### Acidification par l'acide citrique.

On met en suspension 61,1 g (0,1 mol) de diosmine à purifier dans 100 ml d'eau distillée. Cette diosmine possède un taux d'iode résiduel de 1350 ppm et un taux de cendres sulfuriques de 0,45% (mêmes méthodes de dosage que pour l'exemple 1).
On ajoute 1,86 g de zinc pulvérulent, soit 3% en poids par rapport à la diosmine à purifier. On ajoute 9,9 g (0,247 mol) de soude en solution dans 148 ml d'eau, soit un excès de 1,235 par rapport aux fonctions phénoliques de la diosmine. L'addition de la solution de soude se fait à 25°C pendant 30 minutes.
Lorsque toute la solution aqueuse de soude est ajoutée, on élève la température à 35°C et on maintient cette température pendant 3 h, tout en agitant le milieu.

On filtre ensuite le milieu réactionnel puis on récupère le filtrat après rinçage du filtre avec 140 ml d'eau.
On introduit par ailleurs dans un réacteur 72,8 g d'une solution aqueuse d'acide citrique à 34% en poids (0,129 mol). On ajoute le filtrat obtenu précédemment. La diosmine précipite dans le réacteur.
On filtre le précipité à 35°C. Le gâteau recueilli sur le filtre est ensuite lavé avec 120 ml d'eau puis réempâté dans 300 ml d'eau à 35°C pendant 1 h puis filtré et lavé à nouveau avec 120 ml d'eau.
La diosmine obtenue est séchée à 50°C sous vide partiel de 10mm Hg.
Après séchage, on obtient 59 g de diosmine, le rendement de purification est de 96,5%.
Elle présente un taux d'iode résiduel de 62 ppm et un taux de cendres sulfuriques de 0,33% (mêmes méthodes de dosage que pour le produit brut).

### Exemples comparatifs

Ces exemples ne font pas partie de l'invention. Ils ont été réalisés dans le but de montrer que le métal utilisé dans le cadre de l'invention ne correspond pas à un choix arbitraire, mais, au contraire, qu'il correspond à une sélection nécessaire pour obtenir l'effet technique recherché.

### Exemple comparatif A : Utilisation d'un alliage nickel/aluminium

*Premiérs étape* : on met en suspension 60,8 g (0,1 mol) de diosmine à purifier dans 100 ml d'eau distillée. Cette diosmine possède un taux d'iode résiduel de 1350 ppm et un taux de cendres sulfuriques de 0,45% (mêmes méthodes de dosage que pour l'exemple 1).

On ajoute 0,6 g d'un alliage nickel/aluminium en poudre, soit 1% en poids par rapport à la diosmine à purifier. On ajoute 9,9 g (0,247 mol) de soude en solution dans 143 ml d'eau, soit un excès de 1,235 par rapport aux fonctions phénoliques de la diosmine. L'addition de la solution de soude se fait à 25-30°C pendant 1,5 h. Lorsque toute la solution aqueuse de soude est ajoutée, on élève la température à 35°C et on maintient cette température pendant 2,5 h, tout en agitant le milieu.

*Deuxième étape* : on filtre ensuite le milieu réactionnel puis on récupère le filtrat après rinçage du filtre avec 140 ml d'eau.

*Troisième étape* : on introduit par ailleurs dans un réacteur 64,5 g d'une solution d'acide acétique à 30% en poids. On ajoute le filtrat obtenu précédemment. La diosmine précipite dans le réacteur.

On baisse ensuite la température à 20°C. On filtre le précipité. Le gâteau recueilli sur le filtre est ensuite lavé avec 120 ml d'eau puis réempâté dans 300 ml d'eau à 35°C pendant 1 h puis filtré et lavé à nouveau avec 120 ml d'eau.

La diosmine obtenue est séchée à 50°C sous vide partiel de 10mm Hg. Elle présente un taux d'iode résiduel de 293 ppm (dosage par ICP.MS).

### Exemple comparatif B : Utilisation du couple nickel de Raney/borohydrure de sodium.

*Première étape* : on met en suspension 60,8 g (0,1 mol) de diosmine à purifier dans 100 ml d'eau distillée. Cette diosmine possède un taux d'iode résiduel de 1350 ppm et un taux de cendres sulfuriques de 0,45% (mêmes méthodes de dosage que pour l'exemple 1).
On ajoute 2 g de nickel de Raney pulvérulent. On ajoute ensuite 0,06 g de borohydrure de sodium et 9,9 g (0,247 mol) de soude en solution dans 148 ml d'eau, soit un excès de 1,235 par rapport aux fonctions phénoliques de la diosmine. L'addition se fait à 25-30°C pendant 1,5 h. Lorsque toute la solution aqueuse est ajoutée, on agite le milieu pendant encore 1,5 h.

Les deuxième et troisième étapes sont identiques à celles décrites dans l'exemple comparatif A.
La diosmine obtenue présente un taux d'iode résiduel de 618 ppm (dosage par ICP.MS)

## Revendications

1. Procédé de purification de la diosmine, **caractérisé en ce que** :
- dans une première étape, dans un milieu aqueux :
* on fait réagir la diosmine à purifier avec une base minérale de façon à transformer la diosmine en phénate soluble dans le milieu réactionnel,
* on ajoute du zinc pulvérulent,
- dans une deuxième étape, on filtre le milieu réactionnel,
- dans une troisième étape, on acidifie le filtrat recueilli dans la seconde étape de façon à faire précipiter la diosmine que l'on récupère ensuite de façon usuelle.

2. Procédé selon la revendication 1, **caractérisé en ce que** la réaction de la diosmine à purifier avec la base minérale a lieu en présence du zinc pulvérulent.

3. Procédé selon la revendication 2, **caractérisé en ce qu'**on mélange le zinc et la solution aqueuse de diosmine puis **en ce qu'**on ajoute la base minérale.

4. Procédé selon la revendication 1, **caractérisé en ce que** la base minérale est choisie dans le groupe constitué par la soude, la potasse et leurs mélanges.

5. Procédé selon la revendication 1, **caractérisé en ce que** la teneur en zinc pulvérulent est comprise entre 0,5% en poids et 5% en poids par rapport à la diosmine à purifier.

6. Procédé selon la revendication 1, **caractérisé en ce que** le temps de réaction lors de la première étape est compris entre 0,5 h et 10 h.

7. Procédé selon la revendication 1, **caractérisé en ce que** la température du milieu réactionnel est comprise entre 0°C et 60°C lors de la première étape et de la troisième étape.

8. Procédé selon la revendication 1, **caractérisé en ce que** lors de la troisième étape, on acidifie le filtrat à l'aide d'un acide de pka ≤ 3.

9. Procédé selon la revendication 8, **caractérisé en ce que** l'acide est choisi dans le groupe constitué par l'acide chlorhydrique, l'acide sulfurique et leurs mélanges.

## Patentansprüche

1. Verfahren zur Reinigung von Diosmin, **dadurch gekennzeichnet, dass**
- in einem ersten Schritt in einem wässrigen Medium:
das zu reinigende Diosmin mit einer anorganischen Base umgesetzt wird, um das Diosmin in das in dem Reaktionsmedium lösliche Phenat umzuwandeln, und Zink in Pulverform zugegeben wird,
- das Reaktionsmedium in einem zweiten Schritt filtriert wird, und
- das in dem zweiten Schritt gewonnene Filtrat in einem dritten Schritt angesäuert wird, um das Diosmin auszufällen, welches anschließend in üblicher Weise gewonnen wird.

2. Verfahren nach Anspruch 1, **dadurch gekennzeichnet, dass** die Umsetzung des zu reinigenden Diosmin mit der anorganischen Base in Gegenwart des Zink in Pulverform durchgeführt wird.

3. Verfahren nach Anspruch 2, **dadurch gekennzeichnet, dass** das Zink und die wässrige Diosmin-Lösung vermischt werden und anschließend die anorganische Base zugegeben wird.

4. Verfahren nach Anspruch 1, **dadurch gekennzeichnet, dass** die anorganische Base unter Natriumhydroxid, Kaliumhydroxid und deren Gemischen ausgewählt ist.

5. Verfahren nach Anspruch 1, **dadurch gekennzeichnet, dass** der Mengenanteil des pulverförmigen Zink im Bereich von 0,5 bis 5 Gew.-%, bezogen auf das zu reinigende Diosmin liegt.

6. Verfahren nach Anspruch 1, **dadurch gekennzeichnet, dass** die Reaktionszeit im ersten Schritt im Bereich von 0,5 bis 10 h liegt.

7. Verfahren nach Anspruch 1, **dadurch gekennzeichnet, dass** die Temperatur des Reaktionsmediums während des ersten Schritts und des dritten Schritts im Bereich von 0 bis 60 °C liegt.

8. Verfahren nach Anspruch 1, **dadurch gekennzeichnet, dass** das Filtrat im dritten Schritt mit einer Säure von pKₐ ≤ 3 angesäuert wird.

9. Verfahren nach Anspruch 8, **dadurch gekennzeichnet, dass** die Säure unter Salzsäure, Schwefelsäure und deren Gemischen ausgewählt ist.

## Claims

1. Process for purifying diosmin, **characterized in that**:
- in a first step, in an aqueous medium:
* the diosmin to be purified is reacted with a mineral base so as to convert the diosmin into the phenoxide, which is soluble in the reaction medium,
* pulverulent zinc is added,
- in a second step, the reaction medium is filtered,
- in a third step, the filtrate collected in the second step is acidified so as to precipitate the diosmin, which is then recovered in the usual manner.

2. Process according to Claim 1, **characterized in that** the reaction of the diosmin to be purified with the mineral base takes place in the presence of pulverulent zinc.

3. Process according to Claim 2, **characterized in that** the zinc and the aqueous diosmin solution are mixed together and the mineral base is then added.

4. Process according to Claim 1, **characterized in that** the mineral base is chosen from the group consisting of sodium hydroxide and potassium hydroxide, and mixtures thereof.

5. Process according to Claim 1, **characterized in that** the pulverulent zinc content is between 0.5% by weight and 5% by weight relative to the diosmin to be purified.

6. Process according to Claim 1, **characterized in that** the reaction time during the first step is between 0.5 hour and 10 hours.

7. Process according to Claim 1, **characterized in that** the temperature of the reaction medium is between 0°C and 60°C during the first step and the third step.

8. Process according to Claim 1, **characterized in that**, during the third step, the filtrate is acidified using an acid with a pKa ≤ 3.

9. Process according to Claim 8, **characterized in that** the acid is chosen from the group consisting of hydrochloric acid and sulfuric acid, and mixtures thereof.
